# EUROPÄISCHE PATENTSCHRIFT

(11) **EP 3 457 915 B1**
(45) Veröffentlichungstag und Bekanntmachung des Hinweises auf die Patenterteilung: **02.07.2025**
(21) Anmeldenummer: 17727811.6
(22) Anmeldetag: 11.05.2017
(51) Int. Cl.: A61B 5/00, A61B 5/22

(54) **VORRICHTUNG ZUR MESSUNG, DIAGNOSTIK UND/ODER THERAPIE DER KRÄFTE DER MENSCHLICHEN FINGER, HAND, ARM UND/ODER SCHULTER UND DEREN VERWENDUNG FÜR MESSUNG UND/ODER TRAINING**
DEVICE FOR MEASUREMENT, DIAGNOSIS AND/OR THERAPY OF THE STRENGTH OF THE HUMAN FINGER, HAND, ARM AND/OR SHOULDER AND ITS USE FOR MEASUREMENT AND/OR TRAINING
DISPOSITIF DE MESURE, DE DIAGNOSTIC ET/OU DE THÉRAPIE DES FORCES DES DOIGTS, DE LA MAIN, DU BRAS ET/OU DES ÉPAULES D'UN ÊTRE HUMAIN ET SON UTILISATION POUR MÉSURER ET/OU PRATIQUER

(30) Priorität: 17.05.2016 DE 102016208443
(43) Veröffentlichungstag der Anmeldung: 27.03.2019
(73) Patentinhaber: Weber, Cornelius, 01662 Meissen (DE); Weber, Constanze Anna Maria, 012777 Dresden (DE)
(72) Erfinder: WEBER, Cornelius, 01662 Meissen (DE)
(74) Vertreter: Rauschenbach, Marion
(86) Internationale Anmeldenummer: PCT/EP2017/061344
(87) Internationale Veröffentlichungsnummer: WO 2017/198543

(56) Entgegenhaltungen:
- EP-A1- 0 495 461
- DD-A1- 204 206
- US-A1- 2012 255 355
- US-B1- 8 082 786
- LIU PU ET AL: "EMG-force estimation for multiple fingers", 2013 IEEE SIGNAL PROCESSING IN MEDICINE AND BIOLOGY SYMPOSIUM (SPMB), IEEE, 7 December 2013 (2013-12-07), pages 1 - 6, XP032566939, DOI: 10.1109/SPMB.2013.6736772
- MILLER L C ET AL: "A Wrist and Finger Force Sensor Module for Use During Movements of the Upper Limb in Chronic Hemiparetic Stroke", IEEE TRANSACTIONS ON BIOMEDICAL ENGINEERING, IEEE SERVICE CENTER, PISCATAWAY, NJ, USA, vol. 56, no. 9, 1 September 2009 (2009-09-01), pages 2312 - 2317, XP011343045, ISSN: 0018-9294, DOI: 10.1109/TBME.2009.2026057

## Beschreibung

Die vorliegende Erfindung bezieht sich auf das Gebiet der Medizintechnik, Medizindiagnostik, Physiotherapie und medizinische Rehabilitation und betrifft eine Vorrichtung zur Messung, Diagnostik und/oder Therapie der Kräfte der menschlichen Finger, Hand, Arm und/oder Schulter, wie sie beispielsweise zur Messung der Beugekräfte der Hand oder des Armes, zur Feststellung von Problemen beim Beugen von Hand oder Arm und zur Behebung dieser Probleme zum Einsatz kommen kann.

Eine Vorrichtung, mit der die Beugekräfte der menschlichen Hand oder des zweiten bis fünften Fingers und der verschiedenen Beugewinkel gemessen werden können, ist aus der DD-PS 204 206 bekannt. Die Messung dieser Kräfte wird dadurch bewirkt, dass die Griffteile auf senkrecht zueinander stehenden Linien frei wählbar sind und ein vom zweiten bis fünften Finger erfasstes, vertikal auf die Höhe jedes Fingers einstellbares Griffteil im Wirkungseingriff mit einem Kraftmessaufnehmer (Messwandler) steht. Das bewegliche Griffteil ist mit einem Zugbolzen verbunden, der an seinem entgegengesetzten Ende einen Flansch aufweist, der unter der Kraft einer Druckfeder am zentralen Bereich der Druckmessdose anliegt, die den Zugbolzen koaxial verkantungsfrei umfasst. Eine fehlerfreie Messung der Beugekraft des Daumens ist jedoch mit dieser Vorrichtung nicht möglich.

Die Messung der Beugekräfte des Daumens wird durch eine Vorrichtung gemäß DE-PS 36 34 940 möglich, bei der unter Verwendung der vorbeschriebenen Konstruktion der horizontale und vertikale Winkel der Längsachse eines Handgriffes mit Widerlage zur Längsachse des mit dem Messwandler im Wirkungseingriff stehenden Zugbolzens und seiner Neigung gegenüber der Horizontalebene in einer zweiten, senkrecht zur ersten befindlichen Vertikalebene durch einen Drehständer und zwei Drehgelenken sowie der Längs- und Querabstand des Handgriffes zu dem mit dem Zugbolzen verbundenen Fingerzuggriff mit Hilfe eines Längs- und eines Quersupports frei einstellbar sind.

Die bekannten Vorrichtungen ermöglichen zwar exakte Messungen der Beugekräfte der menschlichen Hand oder einzelner Finger jeweils genau in Wirkungsrichtung der Kraft bei biomechanisch gleichen Bedingungen, für Hände unterschiedlicher Größe und bei verschiedenen Beugewinkeln der Finger ein und derselben Hand, gestatten sie jedoch nicht die Messung der für die verschiedenen Gebiete der Medizin bedeutsamen Streckkräfte der menschlichen Hand oder ihrer einzelnen Finger.

Aus der US 3 680 386 A ist eine Messvorrichtung bekannt, mit der für Körperglieder die Größe einer Stoßkraft und auch einer Zugkraft gemessen werden kann.

Auch ist gemäß EP 0 495 461 A1 eine Vorrichtung zum Messen der Kräfte der menschlichen Hand oder ihrer einzelnen Finger mittels eines Kraftmessaufnehmers bekannt, bei der der Kraftmessaufnehmer einen ihn durchdringenden Zugbolzen umfasst und von einem Gehäuse umgeben ist, wobei eine bei mit einem Betätigungsteil aufgebrachten Beugekräften oder Zugkräften sich am Gehäuse und eine bei mit einem Betätigungsteil aufgebrachten Streckkräften oder Druckkräften sich am Kraftmessaufnehmer abstützende Reversierscheibe vorgesehen ist.

Kraftmessaufnehmer sind Kraftaufnehmer mit Messfunktion. Kraftaufnehmer werden auch Kraftsensoren genannt, wobei die Kraft gemessen wird, die auf einen Sensor wirkt. Allgemein bekannte Kraftmessaufnehmer sind Federkörper-Kraftaufnehmer, Piezo-Kraftaufnehmer, Kraftaufnehmer mit schwingenden Elementen, Kraftaufnehmer mit elektromagnetischer Kompensation und resistive Kraftaufnehmer (Wikipedia Stichwort Kraftaufnehmer).

Weiterhin ist aus der US 2012/255355 A1 eine Vorrichtung zur Messung der Kräfte und Fingerfertigkeit der Hand bekannt, die einen Kraftsensor enthält, der in einem vorderen Griff positioniert ist.

Nach der US 8,082,786 B1 ist ein tragbares Gerät zur Prüfung der Arbeitskapazität bekannt, welches einen tragbaren Computer aufweist, der mit einer dynamischen Kraftmess- und Hebevorrichtung über eine Nabe lösbar verbunden ist, wobei mit der Nabe eine Reihe von anderen Geräten und Testvorrichtungen lösbar verbunden werden können. Diese weiteren Geräte und Testvorrichtungen können Vorrichtungen zur Prüfung der Fingerklemmkraft, der Fingerbeugekraft, der Handgriffkraft, der Unterarmkraft, der Handgelenkkraft, aber auch zur Prüfung der Koordination des gesamten Körpers sein.

Nachteilig an den bekannten technischen Lösungen ist, dass keine multifunktionelle Messung, Diagnostik und/oder Therapie der Kräfte der menschlichen Finger, Hand, Arm und/oder Schulter an einer Vorrichtung möglich ist.

Die Aufgabe der Erfindung besteht in der Angabe einer Vorrichtung zur Messung, Diagnostik und/oder Therapie der Kräfte der menschlichen Finger, Hand, Arm und/oder Schulter, mit deren Hilfe eine multifunktionelle Messung, Diagnostik und/oder Therapie der Kräfte der menschlichen Finger, Hand, Arm und/oder Schulter an einer Vorrichtung möglich ist.

Die Aufgabe wird durch die in den Ansprüchen angegebene Erfindung gelöst. Vorteilhafte Ausgestaltungen sind Gegenstand der Unteransprüche, wobei die Erfindung auch Kombinationen der einzelnen abhängigen Ansprüche im Sinne einer und-Verknüpfung mit einschließt, solange sie sich nicht gegenseitig ausschließen.

Die erfindungsgemäße Vorrichtung zur Messung, Diagnostik und/oder Therapie der Kräfte der menschlichen Finger, Hand, Arm und/oder Schulter besteht mindestens aus einer Grundplatte und Kraftmessaufnehmern, von denen mindestens einer einen ihn durchdringenden Zugbolzen aufweist und dieser Kraftmessaufnehmer an einer Seite der Grundplatte ortsfest positioniert ist und dieser Kraftmessaufnehmer über den Zugbolzen kraftschlüssig mit einem Betätigungsteil verbunden ist, weiterhin an der dem ortsfest positionierten Kraftmessaufnehmer gegenüberliegenden Seite der Grundplatte und mindestens teilweise im Bereich der an dieser Seite anschließenden Seiten der Grundplatte eine Führung vorhanden ist, auf oder an der mindestens ein beweglicher Kraftmessaufnehmer positioniert ist.

Vorteilhafterweise sind die Kraftmessaufnehmer der erfindungsgemäßen Vorrichtung Federkörper-Kraftaufnehmer, Piezo-Kraftaufnehmer, Kraftaufnehmer mit schwingenden Elementen, Kraftaufnehmer mit elektromagnetischer Kompensation oder Dehnungsmessstreifen-Kraftaufnehmer.

Auch ist vorteilhafterweise als Betätigungsteil der erfindungsgemäßen Vorrichtung ein Fingergriffteil, ein Handgriffteil, eine Handstütze, eine Daumenstütze oder eine Ellenbogenschale vorhanden, wobei diese Betätigungsteile für die einzelnen Finger für die rechte oder linke Hand, Arm oder Schulter geformt sind.

Noch vorteilhafterweise sind bei dem Handgriffteil Vorrichtungselemente vorhanden, die die ortsfeste Positionierung der Finger während der Messung und/oder Therapie realisieren.

Vorteilhafterweise sind als Vorrichtungselemente lösbare Verbindungselemente, Bolzen, Feststellschrauben oder Griffmulden vorhanden.

Die kraftschlüssige Verbindung zwischen Zugbolzen und Betätigungsteil ist vorteilhafterweise über einen Schnellverschluss oder Feststellschraube realisiert.

Ebenfalls ist vorteilhafterweise als Grundplatte eine rechteckige Platte, die aus Holz oder Kunststoff oder Metall mit oder ohne Polsterelementen ist, eingesetzt.

Noch vorteilhafterweise ist die Grundplatte im Querschnitt über ihre Länge an eine Unterarm- oder Handform angepasst oder anpassbar.

Weiterhin ist vorteilhafterweise an der dem ortsfest positionierten Kraftmessaufnehmer gegenüberliegenden Seite der Grundplatte die Führung in Form einer U-förmigen Metallschiene am Rand der Grundplatte oder in Form einer Nut am äußeren Rand der Grundplatte vorhanden.

Und auch vorteilhafterweise dient die Führung zur Aufnahme einer Befestigungsvorrichtung für einen oder mehrere Kraftmessaufnehmer, wobei diese verschiebbar und fixierbar auf oder an der Führung angeordnet sind.

Ebenfalls vorteilhafterweise sind axial und/oder radial bewegliche Hilfsvorrichtungen an und/oder auf der Grundplatte vorhanden, die zur Positionierung der Finger, der Hand und/oder des Armes und/oder als Widerlager dienen.

Vorteilhafterweise sind die axial beweglichen Hilfsvorrichtungen zur Positionierung oder als Widerlager für die Messung und/oder Training der Finger, der Hand und/oder des Armes vorhanden.

Auch vorteilhafterweise sind die radial beweglichen Hilfsvorrichtungen zur Positionierung oder als Widerlager für die Messung und/oder Training der Finger, der Hand, des Armes oder der Schulter vorhanden.

Ebenfalls vorteilhafterweise ist als Hilfsvorrichtungen eine Handstütze, eine Daumenstütze oder eine Ellenbogenschale vorhanden, wobei diese Hilfsvorrichtungen für die rechte oder linke Hand, rechten oder linken Daumen oder rechter oder linker Ellenbogen geformt sind.

Vorteilhafterweise sind als Hilfsvorrichtungen Fixierelemente für die Finger, die Hand, den Arm oder den Ellenbogen vorhanden.

Auch ist vorteilhafterweise als Hilfsvorrichtungen eine im Bereich des ortsfesten Kraftmessaufnehmers fixierte Haltevorrichtung für einen weiteren Kraftmessaufnehmer positioniert, wobei diese Haltevorrichtung einen weiteren Kraftmessaufnehmer rechts und/oder links von der Grundplatte im Bereich des Daumens oder der Hand positioniert und für die Kraftaufnahme von Daumenkräften oder Drehkräften der Hand oder des Unterarmes ausgeführt ist.

Die erfindungsgemäße Verwendung der erfindungsgemäßen Vorrichtung zur Messung und Training, oder Training der Beuge-, Zug-, Streck-, Dreh- und/oder Druckkräfte der menschlichen Finger, Hand, Arm oder Schulter, wobei vorteilhafterweise die Kraft der Muskulatur von Finger, Hand, Arm oder Schulter gemessen und trainiert oder trainiert wird, und wobei vorteilhafterweise die Kraft von Bizeps, Trizeps oder Deltamuskel gemessen und trainiert oder trainiert wird.

Mit der vorliegenden Erfindung ist es erstmals möglich, eine Vorrichtung anzugeben, bei der die Messung, Diagnostik und/oder Therapie der Kräfte der menschlichen Finger, Hand, Arm und/oder Schulter an einer Vorrichtung möglich ist.

Ausgehend von der an sich bekannten Vorrichtung zum Messen der Kräfte der menschlichen Hand oder ihrer einzelnen Finger gemäß EP 0 495 461 A1 ist erfindungsgemäß die Vorrichtung dahingehend erweitert worden, dass sie nicht nur zur Messung, sondern auch zur Diagnostik und/oder Therapie der Kräfte, sondern dass mit ihr auch die Messung, Diagnostik und/oder Therapie der Kräfte des Armes und/oder der Schulter realisiert werden kann.

Die an sich bekannte Vorrichtung besteht aus einem Kraftmessaufnehmer, der einen ihn durchdringenden Zugbolzen umfasst und von einem Gehäuse umgeben ist, wobei ein Betätigungsteil zur Aufbringung von Beugekräften oder Zugkräften kraftschlüssig mit dem Zugbolzen verbunden ist, wobei eine Handstütze als ein Widerlager zur Aufbringung von Beugekräften und Zugkräften vorgesehen ist, wobei ein Betätigungsteil zur Aufbringung von Streckkräften oder Druckkräften kraftschlüssig mit einer Reversierscheibe verbunden ist, wobei ein Ellbogenwiderlager zur Aufbringung von Streckkräften oder Druckkräften vorgesehen ist, wobei sich die Reversierscheibe bei aufgebrachten Beugekräften oder Zugkräften am Gehäuse abstützt, wobei der Zugbolzen in axialer Richtung relativ zu der Reversierscheibe und dem Kraftmessaufnehmer verschiebbar ist, und wobei bei aufgebrachten Streckkräften oder Druckkräften am Kraftmessaufnehmer abgestützt, wobei die Reversierscheibe und der Kraftmessaufnehmer relativ zu dem Zugbolzen und dem Gehäuse verschiebbar sind.

Darauf aufbauend können mit der erfindungsgemäßen Vorrichtung nunmehr die Kräfte des gesamten Armes einschließlich der Schulter in verschiedener radialer oder axialer Richtung sowohl am rechten als auch am linken Arm einerseits gemessen, andererseits Probleme diagnostiziert und diese dann auch therapiert werden.

Die zu gewinnenden Kraftwerte können in Verbindung mit anthropometrischen Messwerten Aussagen über die physische Entwicklung von Menschen ermöglichen. Sie können als normative Grundlage für die Bewertung der Leistungsfähigkeit und des Gesundheitszustandes der Menschen herangezogen werden, die an Erkrankungen leiden, welche die Funktion des Schultergelenkes beeinträchtigen.

Die Zunahme der Schulterkraft ist Ursprung für die weiteren Verbesserungen der Leistungsfähigkeit der Schulter. Durch ein gezieltes Training kommt es zum Kraftanstieg der Schultermuskulatur. Die gestärkten Muskeln können nun die Gelenkpfanne und -kapsel entlasten. So werden folgende Abnutzungserscheinungen reduziert und eventuelle Entzündungsursachen oder -herde beseitigt. Dies ist wiederum bewegungsfördernd und ermöglicht einen sich weiter verbessernden Trainingseinsatz. Die Kraft nimmt weiter zu und die Mobilität der Schulter und des ganzen Armes und der Hand verbessert sich nach und nach. Der Trainingserfolg wird somit durch die Kraftzunahme ausgelöst. Das Resultat daraus ist die wieder gewonnene Lebensqualität, welche durch den gesteigerten Einsatzbereich des Armes schlussfolgernd ist.

Mit der erfindungsgemäßen Lösung können sowohl Zug- als auch Druckkräfte und damit sowohl die Beuge- als auch die Streck-, Dreh- und Druckkräfte der menschlichen Hand oder einzelnen Finger, des Arms oder der Schulter genau und mit vergleichsweise geringem Aufwand gemessen, diagnostiziert und therapiert und somit trainiert werden. Die Anwendung der Vorrichtung ist dabei nicht allein auf medizinische Aufgaben beschränkt. Die Vorrichtung kann auch in anderen technischen Systemen eingesetzt werden, wo Kräfte in entgegengesetzten Richtungen gemessen werden müssen.

Besonders vorteilhafterweise ist bei der erfindungsgemäßen Vorrichtung, dass die Vorrichtung mit geringen Aufwand an die Anatomie des jeweiligen menschlichen Körpers angepasst werden kann und die verschiedenen angegebenen Mess-, Diagnostizier- und Therapiemöglichkeiten umsetzbar sind.

Damit eine möglichst genaue Messung vorgenommen werden kann, sind die Kraftmessaufnehmer als Sensoren mit möglichst geringen Berührungsflächen konzipiert und durch ihre Positionierung an der erfindungsgemäßen Vorrichtung werden durch Reibung bedingte Messungenauigkeiten gering gehalten.

Dabei besteht die erfindungsgemäße Vorrichtung mindestens aus einer Grundplatte, die vorteilhafterweise rechteckig geformt und aus Holz oder Kunststoff oder Metall mit oder ohne Polsterungen für die aufliegenden Teile des menschlichen Armes oder der Hand sind. Diese Grundplatte kann ebenfalls im Querschnitt über ihre Länge an die Unterarm- und/oder Handform des menschlichen Körpers angepasst sein oder an die jeweils konkrete Unterarm oder Handform des Patienten anpassbar sein.

Auf oder an der Grundplatte können auch Hilfsvorrichtungen angebracht sein. Wie beispielsweise eine Handstütze, eine Daumenstütze oder eine Ellenbogenschale, wobei diese Hilfsvorrichtungen für die rechte oder linke Hand, rechten oder linken Daumen oder rechter oder linker Ellenbogen geformt sein können. Diese Hilfsvorrichtungen dienen als Fixierelemente für die Finger, die Hand, den Arm oder den Ellenbogen oder als Widerlager.

Diese erfindungsgemäß einsetzbaren Hilfsvorrichtungen können anatomisch geformte Elemente sein, die bei Messung, Diagnostik und/oder Therapie für die Positionierung oder Fixierung oder als Widerlager dienen.

Die Befestigung der Hilfsvorrichtungen erfolgt dabei mittels lösbarer Verbindungen, Bolzen, Feststellschrauben oder einem Schnellverschluss.

Die Kraftmessaufnehmer der erfindungsgemäßen Vorrichtung können Federkörper-Kraftaufnehmer, Piezo-Kraftaufnehmer, Kraftaufnehmer mit schwingenden Elementen, Kraftaufnehmer mit elektromagnetischer Kompensation oder Dehnungsmessstreifen-Kraftaufnehmer sein.

Die erfindungsgemäße Vorrichtung weist weiter mindestens einen Kraftmessaufnehmer auf, der ortsfest an einer Seite der Grundplatte positioniert ist, und einen ihn durchdringenden Zugbolzen, der wiederum kraftschlüssig mit einem Betätigungsteil verbunden ist.

Als Betätigungsteil können ein Fingergriffteil, ein Handgriffteil, eine Handstütze, eine Daumenstütze oder eine Ellenbogenschale vorhanden sein, wobei die Form der Betätigungsteile anatomisch dem menschlichen Körper angepasst sein kann, beispielsweise für die einzelnen Finger für die rechte oder linke Hand, Arm oder Schulter.

Dabei kann das Betätigungsteil ein Fingergriffteil, ein Handgriffteil, eine Handstütze, eine Daumenstütze oder eine Ellenbogenschale sein.

Auch Die Befestigung des Betätigungsteiles kann dabei mittels lösbarer Verbindungen, Bolzen, Feststellschrauben oder einem Schnellverschluss erfolgen.

Das Betätigungsteil kann vorteilhafterweise mit einem Fingergriffteil kombiniert und mit einer Handstütze als Hilfsvorrichtung eingesetzt werden. Die Handstütze dient dazu, dass die Kraft nur vom Finger ausgeht und weitere Kräfte von Hand, Arm oder dem gesamten menschlichen Körper nicht gemessen werden.

Ebenso kann eine Handstütze in Kombination mit einer Ellenbogenschale als Hilfsvorrichtung verwendet werden, um den Unterarm für die Messung der Schulterkraft in Position zu halten.

Wird die Kraft der Hand gemessen, diagnostiziert und/oder therapiert, so wird vorteilhafterweise ein Handgriff verwendet, der die Finger der Hand ortsfest positioniert, so dass die Finger während der Messung, Diagnostik und/oder Therapie nicht zusammenrutschen und aufeinander drücken können.

Um diese ortsfeste Positionierung der Finger an dem Handgriff zu ermöglichen, können lösbare Verbindungselemente, Bolzen, Feststellschrauben oder Griffmulden vorhanden sein, oder der Handgriff kann einen Bogen aufweisen, der der Handform mit gebogenen Fingern ähnelt, oder der Handgriff kann von beiden Seiten der Finger(auf der Seite des Zeigefingers und des kleinen Fingers) mit Begrenzungseinrichtungen auf die Breite der gesamten Finger ortsfest einstellbar sein.

Mit der erfindungsgemäßen Vorrichtung ist es auch möglich, die Kraft des Daumens zu messen. Ebenfalls lässt sich vorteilhafterweise der Handgriff auch schräg fixieren, so dass eine Messung, Diagnostik und/oder Therapie auch bei Menschen durchgeführt werden kann, deren Bewegung des Daumens oder der Hand oder des Armes eingeschränkt sind.

Weiterhin weist die Grundplatte erfindungsgemäß eine Führung für mindestens einen weiteren Kraftmessaufnehmer auf, um die multifunktionelle Messung, Diagnostik und/oder Therapie der verschiedenen Kräfte an Finger, Hand, Arm oder Schulter durchzuführen zu können.

Die Führung ist dabei an der dem ortsfest positionierten Kraftmessaufnehmer gegenüberliegenden Seite der Grundplatte und mindestens teilweise im Bereich der an dieser Seite anschließenden Seiten der Grundplatte vorhanden.

Dabei kann die Führung durch eine U-förmige Metallschiene oder Nut jeweils am äußeren Rand der Grundplatte realisiert sein. Diese Führung nimmt eine Befestigungsvorrichtung für mindestens einen weiteren Kraftmessaufnehmer auf, wobei diese Kraftmessaufnehmer erfindungsgemäß auf oder an der Grundplatte verschoben und fixiert werden können. Dadurch ist der Kraftmessaufnehmer auf und/oder an der Grundplatte geführt und ist so an mehreren Positionen entlang der U-förmigen Metallschiene oder Nut verschieb- und fixierbar, um die verschiedenen Kräfte an Finger, Hand, Arm oder Schulter messen zu können.

So kann der Kraftmessaufnehmer um den Unterarm herumgeführt werden. Das bedeutet genauer, dass weder die Vorrichtung gedreht werden, noch der Patient die Sitzposition bei Messungen aller Schulterkräfte an einem Arm ändern muss. Um das Problem der Kraftableitung bei der Anteversionskraftmessung zu umgehen, kann der bisher eingesetzte Knauf durch einen ergonomisch optimalen Holzgriff ersetzt werden, welcher auf eine Halterung aufgesteckt werden kann, sodass die direkte gradlinige Krafteinleitung in den Kraftmessaufnehmer erfolgen kann. Der neue Holzgriff ist in verschiedenen Größen vorhanden, sodass der Einsatz von Holzgriffen unterschiedlicher Größe für verschieden große Hände möglich ist. Somit sind genauere Messwerte erreichbar, da nun nahezu die gesamte Kraft ungeteilt auf den Kraftmessaufnehmer übertragen werden kann. Bei der Retroversions- und Abduktionskraftmessung kommt der zweite Kraftmessaufnehmer zum Einsatz. Hierbei wird bei der Abduktionsmessung das am Unterarm angreifende Drehmoment auf den Oberarm beseitigt, da der Kraftmessaufnehmer präzise am gewünschten Messpunkt des Oberarmes, dem Epicondylus radialis angesetzt wird. Bei der Retroversion wird statt der bisherigen halbrunden, konkaven Ellenbogen-Holzschale eine ergonomisch an das distale Ende des Oberarmes angepasste Holzformschale als Hilfsvorrichtung verwendet, die eine Krafteinleitung im Bereich der Epicondylen (epicondylus radialis et epicondylus ulnaris) ermöglicht. Durch die erfindungsgemäße Vorrichtung wird eine punktgenaue direkte Krafteinleitungen in die beiden Kraftmessaufnehmer erreicht.

Eine standardisierte Krafteinleitung bei unterschiedlichen Armmaßen wird zum Einen dadurch ermöglicht, dass die Position des verschiebbaren Kraftmessaufnehmers einmal in vertikaler Richtung und zweimal in horizontaler Richtung verstellt werden kann. Zum Anderen erfolgt eine weitere ergonomische Anpassung, indem wesentlich besser als bisher an die Armform angepasste Kraftmessaufnahmeelemente in verschiedenen Armgrößen für die Messung der Kräfte bei Abduktion und Retroversion verwendet werden können. Außerdem stehen verschiedene Griffgrößen für verschiedene Handgrößen zur Messung der Kräfte bei Anteversion zur Verfügung.

Zur Standardisierung der Messung müssen biomechanisch gleiche Messbedingungen für Arme unterschiedlicher Länge geschaffen werden, indem die Kraftübertragung auf das Messsystem im Gegensatz zur bisherigen Vorgehensweise am gleichen anatomischen Punkt erfolgt. Als anatomischer Bezugspunkt wurde der Epicondylus radialis für die Kraftübertragung zur Messung der Armkräfte bei Abduktion ausgewählt. Zur Messung der Armkräfte bei Retroversion ist die äußere Linie am Oberarm vom Epicondylus radialis zum Epicondylus ulnaris die anatomische Bezugslinie für die Kraftübertragung.

Die Positionierung des beweglichen Kraftmessaufnehmers besteht aus der U-förmigen Führung mit einem horizontal stufenlos beweglichen Schlitten. Die vertikale Verstellbarkeit kann dadurch realisiert werden, dass der Schlitten aus zwei rechtwinklig miteinander verbundenen Metallleisten besteht, mit dessen vertikalem Schenkel der Kraftmessaufnehmer beweglich verbunden ist. Der Träger kann mit Hilfe eines Bolzens an der U-förmigen Führung fixiert werden, um um den aufgelegten Arm stufenlos herumgeführt und fixiert werden zu können. Der bewegliche Kraftmessaufnehmer ist in jeder frei wählbaren Position zu beiden Seiten und am Ende der Grundplatte hinter dem Ellenbogen fest verschraubbar. Auf diese Weise kann der Kraftmessaufnehmer zweimal in horizontaler Richtung und einmal in vertikaler Richtung eingestellt werden.

Ein weiterer beweglicher Kraftmessaufnehmer kann auf einer im Bereich des ortsfesten Kraftmessaufnehmers fixierten Haltevorrichtung positioniert sein, wobei die Haltevorrichtung doppelwinkelförmig ausgeführt sein kann und von der linken auf die rechte Seite der Grundplatte schwenkbar angebracht sein kann. Mit dieser Haltevorrichtung kann der weitere bewegliche Kraftmessaufnehmer die Messung, Diagnostik und/oder Therapie von Daumenkräften der rechten oder linken Hand realisieren. Ebenfalls ist die Messung, Diagnostik und/oder Therapie der Drehkräfte der Hand oder des Unterarmes mithilfe eines als weitere Hilfsvorrichtung eingesetzten Griffes möglich, der die Hand oder den Unterarm in verschiedenen Winkeln positioniert und Kräfte auf den Kraftmessaufnehmer ausübt.

Die erfindungsgemäße Vorrichtung zur Messung, Diagnostik und/oder Therapie der Kräfte der menschlichen Finger, Hand, Arm oder Schulter wird zur Messung und Training oder Training der Beuge-, Zug-, Streck-, Dreh- oder Druckkräfte der menschlichen Finger, Hand, Arm oder Schulter verwendet.

Dabei erfolgt der Einsatz der Vorrichtung bei einem Patienten vorteilhafterweise in zwei großen Abschnitten. Jede ist gekennzeichnet durch mehrfachen Wechsel von Kraftausübung und Ruhepause, wobei die Ruhepausen in dem zweiten großen Abschnitt deutlich kürzer sind als im ersten. Mit fortschreitender Kraftzunahme werden die Ruhepausen im weiteren Behandlungsverlauf verkürzt. Vor Beginn der gesamten Trainings- oder Übungsbehandlung wird das Ausgangsniveau der Handschlusskraft links und rechtsseitig dokumentiert, um den Ausgangszustand der Kraftleistung zu erfassen. Sofern nur eine Hand gelähmt ist, kann die Leistung der gesunden Hand als Zielsetzung für die erkrankte Hand dienen. Hierbei muss beachtet werden, ob der Patient Links- oder Rechtshänder ist. Die Primärhand muss eine um circa fünf bis zehn Prozent höhere Handschlusskraft vorweisen als die Sekundärhand. Dies muss bei der Trainingszielsetzung berücksichtigt werden. Abhängig von den Kraftdefiziten werden alle einzelnen Finger oder nur ein Teil jeweils am Mittel- und Endglied behandelt. Jedoch wird fast immer, in Kombination mit der Schwerpunktsetzung auf einzelne Finger die Handschlusskraft der Mittel- und oft auch der Endglieder trainiert. Es ist zu sagen, dass die Behandlung entsprechend dem Schädigungsbild an jeden Patienten spezifisch angepasst wird. Das Training erfolgt meist zweimal pro Woche mit einer Gesamtdauer von jeweils 45 bis 60 Minuten bei Übung oder Training von fünf bis sechs Einzelfunktionen. Die Trainingsintensität wird immer an den Leistungsstand des Patienten angepasst. Um die optimale Kraftentfaltung zu gewährleisten, wird das Gerät mit Schraubzwingen am Tisch fixiert. Der Patient sitzt parallel zum Gerät in aufrechter Körperhaltung auf einem nicht rollenden und höhenverstellbaren Stuhl. Dieser Stuhl wird möglichst so eingestellt, dass der Patient seinen Arm nahezu in der Neutralnullstellung in der Unterarmschale liegen hat und die Griffteile umfassen kann.

Vorteilhafterweise wird die Kraft der Muskulatur von Finger, Hand, Arm oder Schulter gemessen und trainiert oder trainiert, wobei vorteilhafterweise die Messung und das Training oder das Training der Kraft von Bizeps, Trizeps und/oder Deltamuskel erfolgt.

Die erfindungsgemäße Vorrichtung mit den Betätigungsteilen ermöglicht eine genaue Messung der Kräfte in den einzelnen Fingern, der Hand, des Arms und/oder der Schulter, insbesondere durch den Einsatz der erweiterten Vorrichtungsbestandteile und Hilfsvorrichtungen.

In Bezug auf die Messung der Kräfte im Schultergelenk ist nunmehr eine genaue Messung der Kräfte am Arm in den drei Bewegungsrichtungen Anteversion, Retroversion und Abduktion mit der erfindungsgemäßen Vorrichtung möglich.

Mit der erfindungsgemäßen Vorrichtung ist die Kraft und Beweglichkeit der einzelnen Finger, der Hand, des Arms oder der Schulter therapierbar.

Nachfolgend wird die erfindungsgemäße Vorrichtung an mehreren Ausführungsbeispielen näher beschrieben.

Dabei zeigt
- Fig. 1: eine schematische Gesamtansicht der erfindungsgemäße Vorrichtung mit einem Handgriffteil als Betätigungsteil

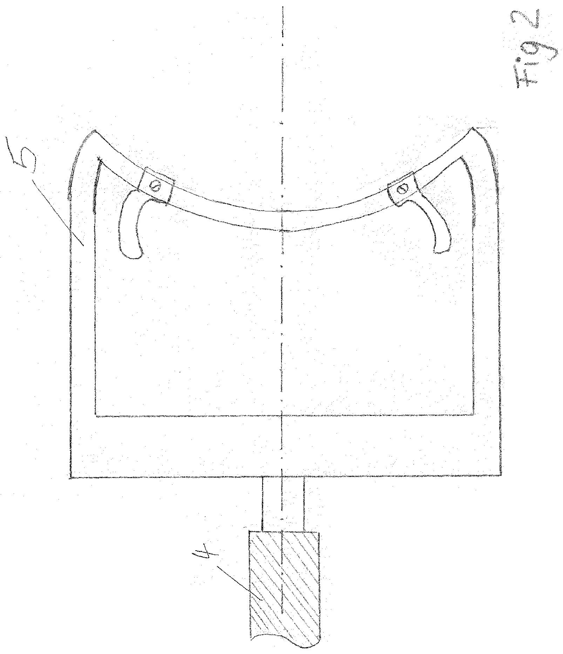

- Fig. 2: eine schematische Detailansicht des vorteilhaften Handgriffteils als Betätigungsteil

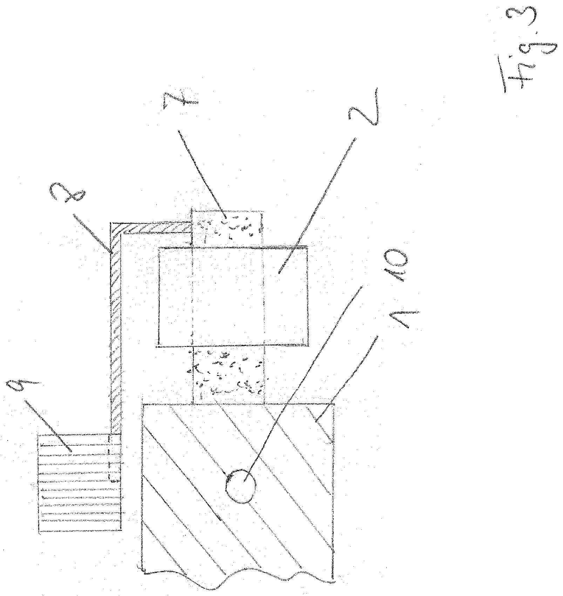

- Fig. 3: schematische Detailansicht der vorteilhaften Anordnung mit einem weiteren Kraftmessaufnehmer zur Messung, Diagnostik und/oder Therapie von Daumenkräften der rechten oder linken Hand und/oder der Drehkräfte der Hand und/oder des Unterarmes mithilfe eines als weitere Hilfsvorrichtung eingesetzten Griffes

### Beispiel 1

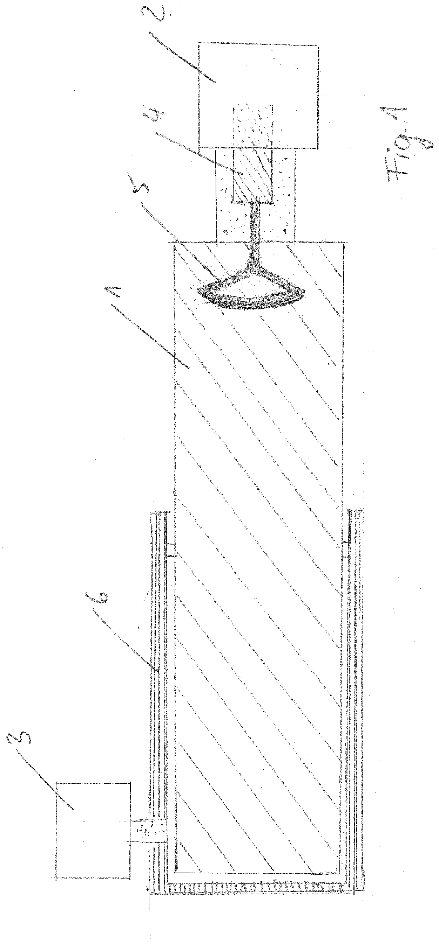

Die Vorrichtung nach Figur 1 weist eine rechteckige Grundplatte 1 aus Holz mit den Abmessungen BxLxH = 60x20x3 cm auf, wobei zwei Kraftmessaufnehmer 2 und 3 an der Grundplatte 1 angeordnet sind. Ein Kraftmessaufnehmer 2 ist an der einen kurzen Seite der rechteckigen Grundplatte 1 über eine Metallverbindung ortsfest positioniert und weist einen ihn durchdringenden Zugbolzen 4 aus Metall auf. Der andere Kraftmessaufnehmer 3 ist mit einer Metallverbindung an der U-förmigen Führung 6 sowohl in axialer als auch in radialer Richtung gegenüber der Grundplatte 1 verschiebbar angeordnet. Die U-förmige Führung 6 ist aus Metall gefertigt und entlang der Längsseiten der rechteckigen Grundplatte 1 jeweils 30 cm geführt und an der Grundplatte 1 befestigt.

Der den ortsfesten Kraftmessaufnehmer 2 durchdringende Zugbolzen 4 ist mit einem Betätigungsteil 5 in Form eines Handgriffes kraftschlüssig verbunden.

Auf der Grundplatte 1 ist als Hilfsvorrichtung ein Unterarmpolster angebracht, auf dem der Unterarm gelagert wird, wobei die Polsterung auch als ortsfeste Fixierung des Unterarmes dient. Zusätzlich ist als Hilfsvorrichtung eine Fixiervorrichtung aus verstellbaren Stangen und Schrauben auf Höhe des Handgelenkes auf der Grundplatte 1 angebracht, die den Unterarm während der Messung/Diagnose/Therapie möglichst unbeweglich fixiert, so dass die Kraft entlang der Achse des Unterarms gemessen werden kann, wenn die Hand ihre Kraft auf den Handgriff ausübt.

Statt des Handgriffteils kann ein Fingergriffteil angeordnet sein. In diesem Fall ist vorteilhafterweise eine Handstütze, beispielsweise ein Bolzen, vorhanden, um die Messung, Diagnostik und/oder Therapie für den Finger durchzuführen und Kräfte ausgehend von Hand, Arm oder dem gesamten menschlichen Körper zu minimieren.

### Beispiel 2

Auf der Grundplatte 1 gemäß Beispiel 1 ist zusätzlich auf der entgegengesetzten Seite des ortsfesten Kraftmessaufnehmers 2 als Hilfsvorrichtung eine in axialer Richtung auf der Grundplatte 1 verschiebbare Ellenbogenschale angebracht, die als Fixierung des Ellenbogens und als Widerlager dient.

Nach Positionierung des Unterarmes auf der Grundplatte 1 und Fixierung des Handgelenkes und des Ellenbogens wird die Messung der Abduktion der Schulter durch Messen der Kraft seitlich am Ellenbogen durch den auf der Führung 6 positionierten Kraftmessaufnehmer 3 durchgeführt, wobei der Kraftmessaufnehmer 3 vorher an die Position des Ellenbogens verschoben worden ist. Auf der Grundplatte 1 befinden sich ein Unterarmpolster und ein Handstütze durch die die Fixierung des Handgelenkes erfolgt. Der Unterarm ist auf der Unterarmstütze positioniert und die Hand umgreift die Handstütze, nun misst der Kraftmessaufnehmer die Kraft der Schulter, wenn mit dem Ellenbogen eine Kraft auf den Kraftmessaufnehmer ausgeübt wird.

### Bezugszeichenliste

- 1: Grundplatte
- 2: Kraftmessaufnehmer
- 3: Kraftmessaufnehmer
- 4: Zugbolzen
- 5: Betätigungsteil
- 6: Führung
- 7: Träger
- 8: Haltevorrichtung
- 9: beweglicher Kraftmessaufnehmer
- 10: Haltegriff als Hilfsvorrichtung

## Patentansprüche

1. Vorrichtung zur Messung, Diagnostik und/oder Therapie der Kräfte der menschlichen Finger, Hand, Arm und/oder Schulter bestehend mindestens aus einer Grundplatte (1) und Kraftmessaufnehmern (2, 3, 9), von denen mindestens einer einen ihn durchdringenden Zugbolzen (4) aufweist und dieser Kraftmessaufnehmer (2) an einer Seite der Grundplatte (1) ortsfest positioniert ist und dieser Kraftmessaufnehmer (2) über den Zugbolzen (4) kraftschlüssig mit einem Betätigungsteil (5) verbunden ist, weiterhin an der dem ortsfest positionierten Kraftmessaufnehmer (2) gegenüberliegenden Seite der Grundplatte (1) und mindestens teilweise im Bereich der an dieser Seite anschließenden Seiten der Grundplatte (1) eine Führung (6) vorhanden ist, auf oder an der mindestens ein beweglicher Kraftmessaufnehmer (3) positioniert ist.

2. Vorrichtung nach Anspruch 1, bei der die Kraftmessaufnehmer (2, 3, 9) Federkörper-Kraftaufnehmer, Piezo-Kraftaufnehmer, Kraftaufnehmer mit schwingenden Elementen, Kraftaufnehmer mit elektromagnetischer Kompensation oder Dehnungsmessstreifen-Kraftaufnehmer sind.

3. Vorrichtung nach Anspruch 1, bei der als Betätigungsteil (5) ein Fingergriffteil, ein Handgriffteil, eine Handstütze, eine Daumenstütze oder eine Ellenbogenschale vorhanden ist, wobei diese Betätigungsteile (5) für die einzelnen Finger für die rechte oder linke Hand, Arm oder Schulter geformt sind, wobei bei dem Handgriffteil Vorrichtungselemente vorhanden sind, die die ortsfeste Positionierung der Finger während der Messung und/oder Therapie realisieren, und wobei als Vorrichtungselemente lösbare Verbindungselemente, Bolzen, Feststellschrauben oder Griffmulden vorhanden sind.

4. Vorrichtung nach Anspruch 1, bei der die kraftschlüssige Verbindung zwischen Zugbolzen (4) und Betätigungsteil (5) über einen Schnellverschluss oder Feststellschraube realisiert ist.

5. Vorrichtung nach Anspruch 1, bei der als Grundplatte (1) eine rechteckige Platte, die aus Holz oder Kunststoff oder Metall mit oder ohne Polsterelementen ist, eingesetzt ist.

6. Vorrichtung nach Anspruch 1, bei der die Grundplatte (1) im Querschnitt über ihre Länge an eine Unterarm- oder Handform angepasst ist oder anpassbar ist.

7. Vorrichtung nach Anspruch 1, bei der an der dem ortsfest positionierten Kraftmessaufnehmer (2) gegenüberliegenden Seite der Grundplatte (1) die Führung (6) in Form einer U-förmigen Metallschiene am Rand der Grundplatte (1) oder in Form einer Nut am äußeren Rand der Grundplatte (1) vorhanden ist.

8. Vorrichtung nach Anspruch 1, bei der die Führung (6) zur Aufnahme einer Befestigungsvorrichtung für einen oder mehrere Kraftmessaufnehmer (3) dient, wobei diese verschiebbar und fixierbar auf oder an der Führung (6) angeordnet sind.

9. Vorrichtung nach Anspruch 1, bei der axial und/oder radial bewegliche Hilfsvorrichtungen an und/oder auf der Grundplatte (1) vorhanden sind, die zur Positionierung der Finger, der Hand, des Armes oder als Widerlager dienen.

10. Vorrichtung nach Anspruch 9, bei der die axial beweglichen Hilfsvorrichtungen zur Positionierung oder als Widerlager für die Messung und/oder Training der Finger, der Hand und/oder des Armes vorhanden sind.

11. Vorrichtung nach Anspruch 9, bei der die radial beweglichen Hilfsvorrichtungen zur Positionierung oder als Widerlager für die Messung und/oder Training der Finger, der Hand, des Armes oder der Schulter vorhanden sind.

12. Vorrichtung nach Anspruch 9, bei der als Hilfsvorrichtungen eine Handstütze, eine Daumenstütze oder eine Ellenbogenschale vorhanden ist, wobei diese Hilfsvorrichtungen für die rechte oder linke Hand, rechten oder linken Daumen, oder rechter oder linker Ellenbogen geformt sind, oder wobei als Hilfsvorrichtungen Fixierelemente für die Finger, die Hand, den Arm oder den Ellenbogen vorhanden sind.

13. Vorrichtung nach Anspruch 9, bei der als Hilfsvorrichtungen eine im Bereich des ortsfesten Kraftmessaufnehmers fixierte Haltevorrichtung für einen weiteren Kraftmessaufnehmer positioniert ist, wobei diese Haltevorrichtung einen weiteren Kraftmessaufnehmer rechts und/oder links von der Grundplatte im Bereich des Daumens oder der Hand positioniert und für die Kraftaufnahme von Daumenkräften oder Drehkräften der Hand oder des Unterarmes ausgeführt ist.

14. Verwendung einer Vorrichtung gemäß Anspruch 1 zur Messung und Training, oder zum Training der Beuge-, Zug-, Streck-, Dreh- oder Druckkräfte der menschlichen Finger, Hand, Arm oder Schulter, wobei vorteilhafterweise die Kraft der Muskulatur von Finger, Hand, Arm oder Schulter gemessen und trainiert oder trainiert wird, und wobei vorteilhafterweise die Kraft von Bizeps, Trizeps oder Deltamuskel gemessen und trainiert oder trainiert wird.

## Claims

1. Device for measuring, diagnosing, and/or therapy of the forces of human fingers, hand, arm, and/or shoulder, comprising at least a base plate (1) and force sensors (2, 3, 9), of which at least one has a tension bolt (4) passing through it, and this force sensor (2) is fixedly positioned on one side of the base plate (1) and is connected friction-locked to an actuating part (5) via the tension bolt (4). Furthermore, on the side of the base plate (1) opposite the fixed force sensor (2) and at least partially in the area of the adjoining sides of the base plate (1), a guide (6) is provided on or in which at least one movable force sensor (3) is positioned.

2. Device according to Claim 1, wherein the force sensors (2, 3, 9) are spring-body force sensors, piezoelectric force sensors, force sensors with oscillating elements, force sensors with electromagnetic compensation, or strain gauge force sensors.

3. Device according to Claim 1, wherein the actuating part (5) is a finger grip part, a hand grip part, a hand support, a thumb support, or an elbow shell, and these actuating parts (5) are shaped for the individual fingers, for the right or left hand, arm, or shoulder, and wherein the hand grip part includes device elements that enable the fixed positioning of the fingers during measurement and/or therapy, and wherein the device elements include detachable connecting elements, bolts, locking screws, or grip recesses.

4. Device according to Claim 1, wherein the force-transmitting connection between the tension bolt (4) and the actuating part (5) is achieved via a quick-release mechanism or a locking screw.

5. Device according to Claim 1, wherein the base plate (1) is a rectangular plate made of wood, plastic, or metal, with or without cushioning elements.

6. Device according to Claim 1, wherein the base plate (1) is adapted or adaptable in cross-section along its length to the shape of a forearm or hand.

7. Device according to Claim 1, wherein on the side of the base plate (1) opposite the fixed force sensor (2), the guide (6) is in the form of a U-shaped metal rail at the edge of the base plate (1) or in the form of a groove at the outer edge of the base plate (1).

8. Device according to Claim 1, wherein the guide (6) serves to accommodate a fixing device for one or more force sensors (3), which are arranged movably and fixably on or in the guide (6).

9. Device according to Claim 1, wherein axially and/or radially movable auxiliary devices are provided on and/or in the base plate (1), which serve for positioning the fingers, hand, arm, or as abutments.

10. Device according to Claim 9, wherein the axially movable auxiliary devices are provided for positioning or as abutments for the measurement and/or training of the fingers, hand, and/or arm.

11. Device according to Claim 9, wherein the radially movable auxiliary devices are provided for positioning or as abutments for the measurement and/or training of the fingers, hand, arm, or shoulder.

12. Device according to Claim 9, wherein the auxiliary devices include a hand support, a thumb support, or an elbow shell, and these auxiliary devices are shaped for the right or left hand, right or left thumb, or right or left elbow, or wherein the auxiliary devices include fixing elements for the fingers, hand, arm, or elbow.

13. Device according to Claim 9, wherein the auxiliary devices include a holding device fixed in the area of the fixed force sensor for an additional force sensor, and this holding device positions an additional force sensor to the right and/or left of the base plate in the area of the thumb or hand and is designed for measuring the forces of the thumb or rotational forces of the hand or forearm.

14. Use of a device according to Claim 1 for measuring and training, or for training the flexion, tension, extension, rotational, or pressure forces of the human fingers, hand, arm, or shoulder, wherein advantageously the force of the muscles of the fingers, hand, arm, or shoulder is measured and trained or trained, and wherein advantageously the force of the biceps, triceps, or deltoid muscle is measured and trained or trained.

## Revendications

1. Dispositif de mesure, de diagnostic et/ou de thérapie des forces des doigts, de la main, du bras et/ou de l'épaule de l'homme, constitué d'au moins une plaque de base (1) et de capteurs de mesure de force (2, 3, 9), dont au moins un présente un boulon de traction (4) qui le traverse, et ce capteur de mesure de force (2) est positionné de manière fixe sur un côté de la plaque de base (1) et ce capteur de mesure de force (2) est relié par adhérence à un élément d'actionnement (5) par l'intermédiaire du boulon de traction (4), en outre, sur le côté de la plaque de base (1) opposé au capteur de mesure de force (2) positionné de manière fixe et au moins partiellement dans la zone des côtés de la plaque de base (1) se raccordant à ce côté, il existe un élément de guidage (6) sur ou contre lequel est positionné au moins un capteur de mesure de force (3) mobile.

2. Dispositif selon la revendication 1, dans lequel les capteurs de force (2, 3, 9) sont des capteurs de force à corps élastique, des capteurs de force piézoélectriques, des capteurs de force à éléments vibrants, des capteurs de force à compensation électromagnétique ou des capteurs de force à jauge de contrainte.

3. Dispositif selon la revendication 1, dans lequel il existe comme pièce d'actionnement (5) une pièce de préhension des doigts, une partie de préhension de la main, un appui pour la main, un appui pour le pouce ou une coque pour le coude, ces pièces d'actionnement (5) étant formées pour les différents doigts pour la main, le bras ou l'épaule droite ou gauche, des éléments de dispositif étant présents pour la partie de préhension de la main, qui réalisent le positionnement fixe des doigts pendant la mesure et/ou la thérapie, et des éléments de liaison amovibles, des boulons, des vis de blocage ou des cavités de préhension étant présents comme éléments de dispositif.

4. Dispositif selon la revendication 1, dans lequel la liaison par adhérence entre le boulon de traction (4) et la pièce d'actionnement (5) est réalisée par l'intermédiaire d'une fermeture rapide ou d'une vis de blocage.

5. Dispositif selon la revendication 1, dans lequel une plaque rectangulaire, en bois ou en matière plastique ou en métal avec ou sans éléments de rembourrage, est utilisée comme plaque de base (1).

6. Dispositif selon la revendication 1, dans lequel la section transversale de la plaque de base (1) est adaptée ou peut être adaptée sur sa longueur à la forme d'un avant-bras ou d'une main.

7. Dispositif selon la revendication 1, dans lequel, sur le côté de la plaque de base (1) opposé au capteur de force (2) positionné de manière fixe, l'élément de guidage (6) est présent sous la forme d'un rail métallique en forme de U sur le bord de la plaque de base (1) ou sous la forme d'une rainure sur le bord extérieur de la plaque de base (1).

8. Dispositif selon la revendication 1, dans lequel l'élément de guidage (6) sert à recevoir un dispositif de fixation pour un ou plusieurs capteurs de force (3), ceux-ci étant disposés de manière à pouvoir être déplacés et fixés sur ou sur le guide (6).

9. Dispositif selon la revendication 1, dans lequel des dispositifs auxiliaires mobiles axialement et/ou radialement sont présents sur la plaque de base (1), qui servent au positionnement des doigts, de la main, du bras ou comme butée.

10. Dispositif selon la revendication 9, dans lequel les dispositifs auxiliaires mobiles axialement sont présents pour le positionnement ou comme butée pour la mesure et/ou l'entraînement des doigts, de la main et/ou du bras.

11. Dispositif selon la revendication 9, dans lequel les dispositifs auxiliaires mobiles radialement sont présents pour le positionnement ou comme butée pour la mesure et/ou l'entraînement des doigts, de la main, du bras ou de l'épaule.

12. Dispositif selon la revendication 9, dans lequel un appui pour la main, un appui pour le pouce ou une coque pour le coude sont présents comme dispositifs auxiliaires, ces dispositifs auxiliaires étant formés pour la main droite ou gauche, le pouce droit ou gauche, ou le coude droit ou gauche, ou dans lequel des éléments de fixation pour les doigts, la main, le bras ou le coude sont présents comme dispositifs auxiliaires.

13. Dispositif selon la revendication 9, dans lequel un dispositif de maintien fixé dans la zone du capteur de force fixe est positionné comme dispositifs auxiliaires pour un autre capteur de force, ce dispositif de maintien positionnant un autre capteur de force à droite et/ou à gauche de la plaque de base dans la zone du pouce ou de la main et étant réalisé pour l'absorption de force des forces du pouce ou des forces de rotation de la main ou de l'avant-bras.

14. Utilisation d'un dispositif selon la revendication 1 pour la mesure et l'entrainement, ou pour l'entraînement des forces de flexion, de traction, d'extension, de rotation ou de compression des doigts, de la main, du bras ou de l'épaule humains, dans laquelle avantageusement la force des muscles du doigt, de la main, du bras ou de l'épaule est mesurée ou entraînée, et dans laquelle avantageusement la force du biceps, du triceps ou du deltoïde est mesurée et entraînée.
